# EUROPEAN PATENT SPECIFICATION

(11) **EP 2 451 750 B1**
(45) Date of publication and mention of the grant of the patent: **14.06.2017**
(21) Application number: 10742597.7
(22) Date of filing: 07.07.2010
(51) Int. Cl.: C02F 3/28, C02F 11/04, B01D 53/04, B01D 53/22, C12M 1/107, C02F 1/44

(54) **SYSTEM AND METHOD FOR PURIFYING AN AQUEOUS ORGANIC MATTER, WASTE AND/OR WASTEWATER UNDER PRESSURE AND METHOD FOR PRODUCING BIOGAS.**
SYSTEM UND VERFAHREN ZUR REINIGUNG EINES WÄSSRIGEN ORGANISCHEN STOFFES, ABFALLSTOFFES UND/ODER VON ABWASSER UNTER DRUCK UND VERFAHREN ZUR HERSTELLUNG VON BIOGAS
SYSTÈME ET PROCÉDÉ DE PURIFICATION SOUS HAUTE PRESSION DES EAUX D ÉGOUT, REJETS ET EAUX USÉES, ET PROCÉDÉ D OBTENTION DE BIOGAZ

(30) Priority: 08.07.2009 NL 1037103
(43) Date of publication of application: 16.05.2012
(73) Proprietor: Zagt Zelf B.v., 8447 DR Heerenveen (NL)
(72) Inventor: ZAGT, Christiaan Emanuel, NL-8447 DR Heerenveen (NL)
(74) Representative: Verdijck, Gerardus
(86) International application number: PCT/NL2010/000105
(87) International publication number: WO 2011/005079

(56) References cited:
- WO-A1-03/042117
- DE-A1- 3 404 405
- DE-A1- 3 709 174
- NL-C1- 1 033 083
- US-A1- 2003 173 291
- US-B1- 6 296 766

## Description

The present invention is related to a system to purify and/or treat organic waste and/or wastewater under high pressure. More in particular, the system relates to a (micro-) biological method to purify and/or treat a similar diluted flow of organic material, waste and/or sewage, especially suitable to generate sustainable energy.

Well known purification installations make use of, amongst others, vessels and/or reactors in which microorganisms are put. These microorganisms purify and/or treat the supplied influents.

The remaining flow can subsequently be processed more easily and cheaper. In practice, anaerobe processes are used.

The treated water/sludge mixture is usually separated though a sediment process. Most conventional water purification processes take place under atmospheric pressure.

US 2003/173291 discloses a multiple stage digester system that utilizes anaerobic microbes to convert organic material into a biogas.

The present invention aims to foresee in a system to purify and/or treat a flow of organic material, waste and/or wastewater under high pressure, to be executed in a more efficient way.

This aim is reached with a system according to new claim 1.

The bioreactor or pressure vessel is supplied with a flow (influent) consisting of a gas, fluid, solid substance or a combination thereof, to be purified and/or treated. Anaerobe conversion of the substance that is to be purified and/or treated takes place in the pressure vessel, supported by microorganisms whereby, amongst others, biogas is formed. For that purpose, the pressure vessel is sealable. This way, auto-generative pressure is generated by formation of gas as a result of fermentation.

At least a part of the biogas, that is produced under relatively high pressure, is to be used to drive the pump to supply the flow that needs to be purified and/or treated.

Through this effective driving of the supply-pump, a closed energetic system is acquired wherein, under anaerobe conditions, nutrients such as C₆H₁₂O₆ are converted in CO₂, CH₄ or methane and energy with which the pressure in the pressure vessel is build.

Thus achieving that no additional energy needs to be supplied in order to convey the flow that needs to be purified and/or treated nor for conversions and pressure-build in the pressure vessel. This leads to an effective and efficient purification/treatment of the watery flow of organic materials such as wastewater. In the system, according to the invention, organic material is converted in a pressure vessel into, amongst others, pressure.

The pressure-build is used to drive a feed pump of the organic material. Additionally, the pressure-build is preferably used for other purposes such as the removal of products from the pressure vessel and/or generating additional energy.

The earlier described closed energetic system, provided with a feed pump driven by biologically built pressure, a continuous or semi-continuous working installation such as a water treatment plant, can be achieved. Hereby it is possible to realize a so-called stand alone' system wherein the necessary energy to supply the organic material, is delivered from the pressure, build up in the pressure vessel.
A further advantage of the system according to the invention is that at a continuous or semi-continuous use, it's possible to maintain a more or less constant work point for the process conditions in the pressure vessel. Hereby the process can be driven most optimally over longer periods of time and in such a way that the efficiency of the system, and therefore the executed process is enhanced.

Next to that, the pressure vessel is, preferably, equipped with a sludge membrane to separate the sludge from the flow that is to be purified and/or treated.

A control system drives the entire process and/or regulates the process, whereby, amongst others, pressure in the pressure vessel can be regulated. For this purpose, valves are preferably placed in the inlet and the, at least, one exhaust manifold of the pressure vessel with which the control system can regulate the pressure in this pressure vessel. Preferably, the pressure vessel includes a first exhaust manifold for the produced biogas and a second one for the remaining water/sludge mixture of the flow to be purified and/or treated.

The system also has energy generating means in order to generate energy by using at least a part of the produced biogas. This gas contains the energy content that, for example, can be used in a next process. An example thereof is combustion of the methane formed as biogas whereby the energy, obtained from the combustion can be used in a next process.
In a system according to the invention, it is remarkable that
the same biogas can be used to generate a second form of energy with the aid of energy generating means.
Hereby the biologically built up pressure in the pressure vessel is used and converted into usable energy in the form of labor.
This results into an efficient purification step of the system according to the invention with which the purifying of an influent, aided by the energy generating means, can be executed efficiently. The biogases that are formed are, preferably, methane, carbon dioxide, hydrogen-thio-carbon-acid and or hydrogen sulphide.
The formation of these gases strongly depends on the composition, the temperature and the pH value of the flow that is to be purified and/or treated.
Additionally, the formation of gas can be influenced by the choice in quantity, type of microorganisms and the conditions applied in the pressure vessel. Hereby it is possible to make an even more efficient use of the system.
Additionally, the formation of gas can be influenced by the pH of the medium in the pressure vessel. The quantity of dissolved carbon dioxide and other gasses, and the thereby applied process conditions influence the pH in the pressure vessel.

Hereby it is possible to make an even more efficient use of the system. In an advantageous, preferred embodiment, according to the present invention, the control system is provided with means of control in order to regulate the flow through the membrane.

Based on the pressure in the pressure vessel, the control system can control the flow through the sludge membrane.
For that purpose, regulation of the in- and outgoing flows of liquids and gasses (influents and effluents) can be employed.
By doing so, it is possible to separate the well-dosed flow that needs to be purified and/or treated, from the sludge in the reactor.
In a further advantageous preferred embodiment, according to the present invention, a selective membrane is installed in order to remove the carbon dioxide.
By choosing a selective gas membrane or membrane filter, it is possible to remove dissolved carbon dioxide (H₂CO₃) from the water and/or gas-phase, present in the pressure vessel. The removal of this carbon dioxide generates more, purer flows in the pressure vessel.
In a further advantageous preferred embodiment according to the present invention, a selective membrane is installed in order to remove thiocarbonacid.
By choosing a selective gas membrane or membrane filter, it is possible to remove dissolved thiocarbonic acid (H₂CSO₂) from the water and/or gas-phase, present in the pressure vessel. Such thiocarbonic acid comes into existence through pressure. The removal of this thiocarbonic acid generates more, purer flows in the pressure vessel.

By realizing increasing pressure, the purity of, for example, methane in the gas phase will rise.
This is caused by the fact that carbon dioxide, hydrogen sulphide and possible combinations thereof such as thiocarbonic acid according to Henrys' Law, dissolve better in a watery liquid phase than methane. This brings about the increase of the caloric value of the produced biogas (methane).
As an additional advantage the possible emission of carbon dioxide is reduced, and additionally, the quantity of hydrogen sulphide and carbon dioxide in the gas phase is also reduced.
The levels of carbon dioxide and hydrogen sulphide, or possible combinations thereof such as hydrogen thiocarbonacid, are increased in the liquid phase. This accumulation enables the separation of these substances from the methane. This leads to a gas phase in this present example, that has a higher methane quantity or purity. The extra pure methane can be used for other purposes, if required.

According to the present invention, the pump comprises a pneumatically driven pump.
By providing a pneumatic pump that is directly put in motion by the biologically generated gas from the pressure vessel, a second form of energy, can be made use of relatively simply in addition to the combustion heat of biogas.
The use of a so-called pneumatic pump has the advantage that it can be directly put in motion through the produced biogas from the pressure vessel, such as methane. Hereby, energy loss resulting from energy conversion is avoided.

Hereby, the efficiency of the entire system increases even further. Preferably the biologically built up, relatively high pressure from the pressure vessel may be, for instance partly used to drive a so-called heat pump. By using the high pressure from the biogas from the highpressure vessel, the yield of the heat pump can be increased. Hereby also the yield of the entire system according to the present invention is further increased.

The system according to the invention is to be equipped with a control system that contains means to match the capacity of the feed pump with the pressure in the vessel. This generates additional efficiency of the system, according to the invention.

In a further, advantageous embodiment according to the present invention, the system contains a gas buffer in order to store the produced biogas.

By incorporating a gas buffer, the energy, by means of biologically generated pressure, can be stored in the pressure vessel of bioreactor, or be stored separately from the pressure vessel or bioreactor. By doing so, the generated energy can be applied efficiently at any desired moment, hereby increasing the efficiency of the system.
- The invention further relates to a method to purify and/or treatment of (an) organic waste and/or sewage according to claim 8

Such working method offers equal effects and advantages as mentioned in the system.
Preferably, at least a part of the, under relatively high pressure produced biogas, is used to drive the flow that is to be purified or treated, in other words, the feed-pump. Herewith a closed energetic system is obtained wherein, under anaerobe conditions, nutrients such as C₆H₁₂O₆ or other organic hydrocarbons, are converted in gases such as carbon dioxide (CO₂) or methane (CH₄) and energy with which pressure in the pressure vessel is build. Thus achieving that only little additional energy needs to be supplied in order to convey the flow that needs purification/treatment, and conversions and pressure-build in the pressure vessel. This leads to
an effective and efficient purification/treatment of the watery flow of organic material, such as wastewater. In addition, the produced biogas is preferably used to generate energy by using the energy generating means. An efficient process is thus realised.
In addition, the invention relates to a working method to
produce a biogas according to claim 9.
Such a working method offers equal effects and advantages as mentioned with the system. In an advantageous, preferable embodiment, the biogas contains methane, which is generated in a relatively simple way.

Hereby the methane can be used in addition to the caloric value of the methane, by converting high pressure into a form of usable energy such as labour or purity of gas.

Additional advantages, characteristics and details of the invention are explained by means of a preferred embodiment, referring to the attached figure, which shows:
- Figure 1: a system according to the invention.
- Figure 2: a schematic simplification of the workings of the system from figure 1; and
- Figures 3-4; experimental results with the system from figure 1

System 1 has a measure and control system 2 to control the gas flows 3, 9, 14 and/or liquid flows 4, 5, 17 through separation membranes 6, 12 powered by auto-generative biologically generated, green pressure. Measure- and control system 2 is specifically aimed to control a highpressure
membrane bioreactor 8. Reactor 8 can purify organic material, waste and/or domestic- or industrial sewage.
Hereby biogas is released and clean water is produced.

System 1 consists of a vessel or tank 8 that contains a watery suspension 10 of microorganisms (the sludge) that are responsible for a biological conversion process. The biological conversion process includes the anaerobe conversion of organic material into methane, other biogases and carbon dioxide.

The produced biogas is cleansed and unwanted components such as carbon dioxide are removed by the application of a gas membrane 6. The treated water-sludge mixture is separated from each other by means of a sludge membrane 12, like in a membrane reactor.

By this separation the biological active mass is kept in the vessel whilst the clean water is separated.
System 1 contains a pressure vessel (the container or the reactor or the tank 10). This pressure vessel 8 withstands up to 100 Bar.
The purifying process takes place in this pressure vessel 8. The microorganisms in the pressure vessel 8 produce biogas through which the pressure increases auto-generatively.
This pressure is also called 'green pressure', a relatively new biological form of energy. System 1 contains a high pressure resistant bacteria culture. Because of the increasing pressure in the closed (sealed) vessel 8, the solubility of carbon dioxide in the water phase, will increase whereby less carbon dioxide ends up in the biogas. The produced biogas has therefore an extraordinary high methane level of 80 to 90 volume %.
Vessel 8 contains a two phase system, on one hand the gas phase 11 consisting of biogases and on the other hand the liquid phase 10, consisting of the water-sludge mixture.
Because of the auto-generatively increasing pressure, a driving force is created with which the produced biogas can be pressed through gas-membrane 6. One of the goals is to separate methane 15 from the unwanted components 13 as well as to regulate the acidity. Also because of the high pressure a driving force occurs through which the water-sludge mixture is pressed through the liquids-membrane 12. Another goal is to separate the clean water from the sludge.

If the partial In CO₂ pressure rises above 73 bar at a temperature above 31°C supercritical CO₂ arises in the water-sludge mixture.

In, amongst others, the outgoing pipe (low pressure side) of both the gas drain 14 as the water-sludge mixture drain 16, valves 18 are installed that block the outgoing flow. In the shown embodiment, valves 18 are, amongst others, controlled by a pressure sensor 7 that registers the pressure in the pressure vessel 8.

If the pressure decreases as a result of the outflow of gas and/or water-sludge mixture, measure and control system 2 makes sure that the outflows are decreased.
When pressure increases again, due to biological pressure buildup, the necessary valves are put open again.
A goal is to keep system 1, at a preset value, of sufficiently high pressure.
In the embodiment shown, the pressure vessel withstands an internal pressure up to 100 Bar. The pressure vessel has a pressure-meter 7, a pH-meter 21, if needed a thermometer and a scheme to control temperature (not shown). The pressure buildup in the pressure vessel is registered by the pressure-meter.
The 'green pressure' is either directly or indirectly used to power a membrane separation process for the gas phase and/or water phase, with the aim to remove unwanted components from either phase. A control device is used to let the gas flow through the gas membrane when the pressure in the pressure vessel rises higher than a certain adjustable value, and prevents the outflow of gas when the pressure drops lower than a certain adjustable value.
Another control device is used to let the water-sludge mixture flow through the sludge membrane 12, if the pressure in the vessel rises to a certain adjustable value, and prevents the outflow of the water-sludge mixture when the pressure drops lower than a certain adjustable value.
Flow 4 (influent) is carried through vessel 20 and pump 22 to inlet 25 of reactor 8. In the embodiment shown pump 22 is, based on efficiency, coupled to motor 24. In above-mentioned way, flow 4 is brought into the reactor. The produced biogas is disposed through exhaust 3, through the pneumatic motor 24 used to drive pump 22, or and through 14 and subsequently through 15, on to the gas-network. The sludge-water mixture is drained through exhaust 16. The 'dewatered' sludge is drained through drain 27. The sludge-water mixture is drained through exhaust 26. Clean, treated water is drained through exhaust 5.
In the embodiment as shown, control device 2 is connected to valves 18, and the sensors in system 1. Herewith the process conditions are preserved and, if necessary, system 1 is driven.
To the embodiment shown, possible process conditions of system 1 are,
for vessel 20, a volume of 5m³ with inflow of 2,5 m3 on a pressure of 1 atm at 12°C a COD of 32,8 g/l, and a pH of 7. Reactor 8 has, for the gas phase 11, a volume of 7 m3 temperature of 30°C and a composition of 2400 mol CH₄ and 1600 mol CO₂. For liquid phase 10 a volume of 36 m3 is available at a Ph of 7 and temperature of 30°C. The pressure in the reactor is within the range of 1 - 70 bar and, for example in case of sewage, within the range of 1 - 30 bar.
Experiments have shown that the carbon dioxide level of the produced biogas is lower compared to biogas production processes via atmospheric pressure. This is caused by the extra solubility of carbon dioxide according to Henry's law in the water phase at high pressure. The liquid phase, at a temperature of 31°C and a partial CO₂ pressure of 73 bar or higher, also contains supercritical CO₂.
The liquids phase is saturated with carbon dioxide.
This carbon dioxide, obtained from pressure reduction, is used to counteract the pollution and/or fouling and/or scaling of membranes 12. A control device 2 is used that closes valve 18 in the outgoing liquids-pipe 5 when the pollution on the membrane rises to a certain level.

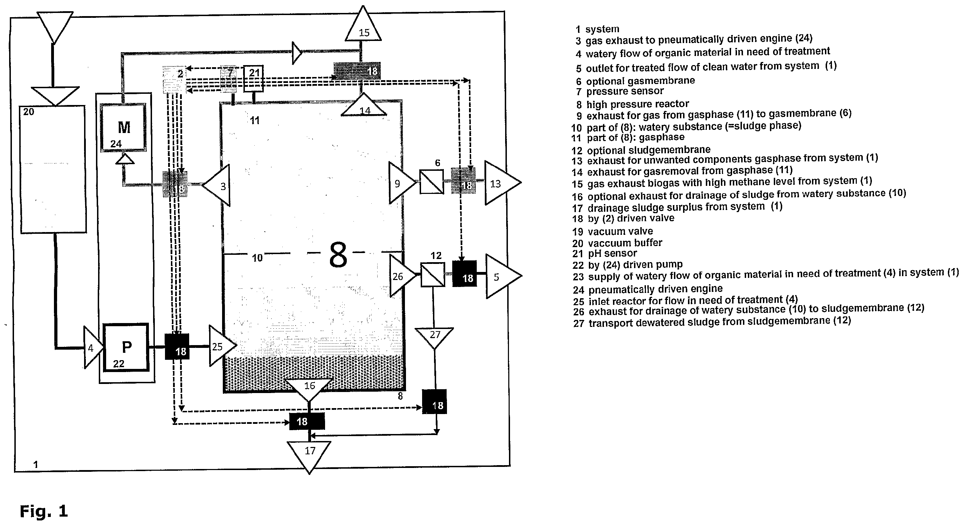

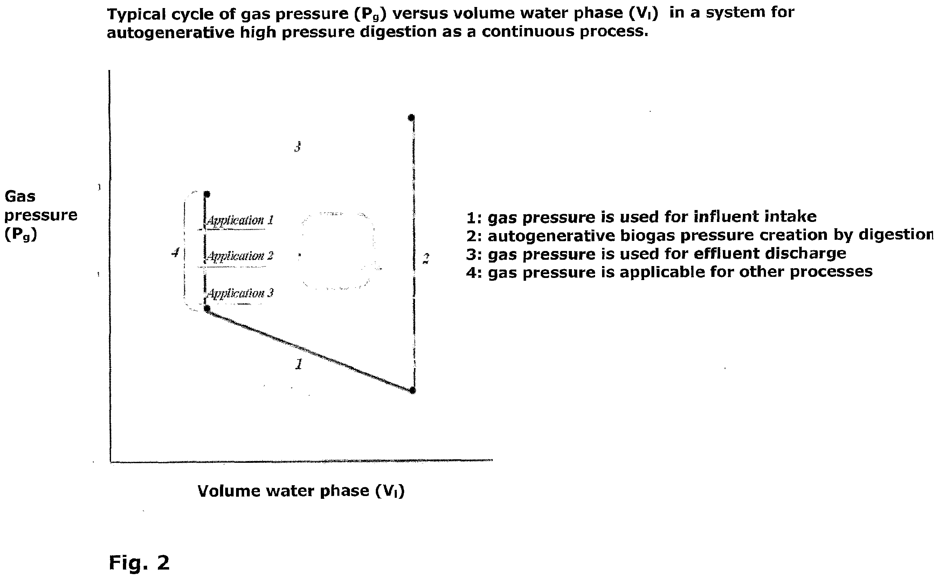

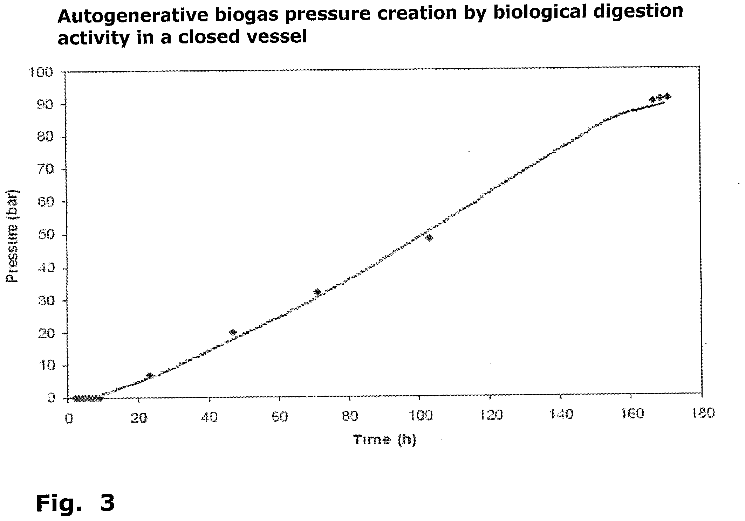

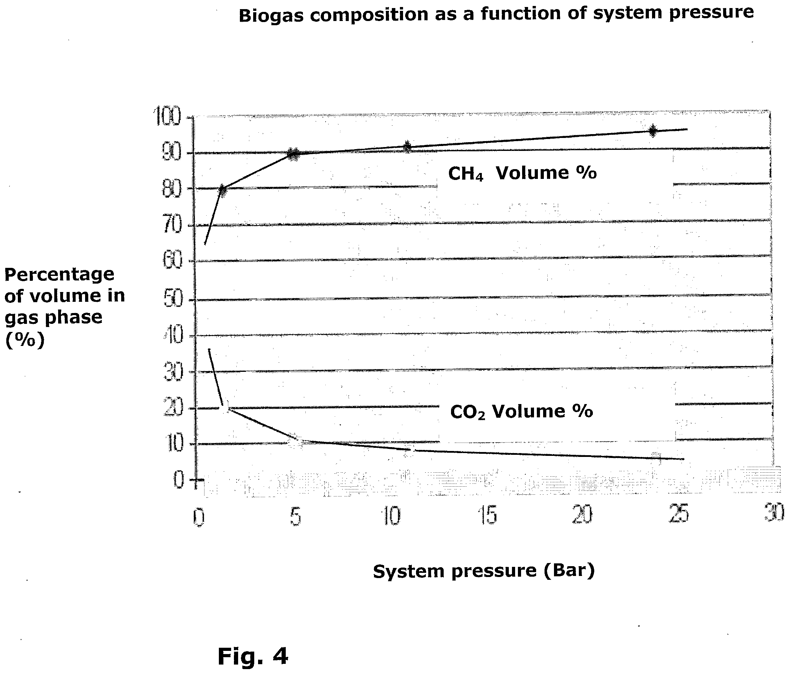

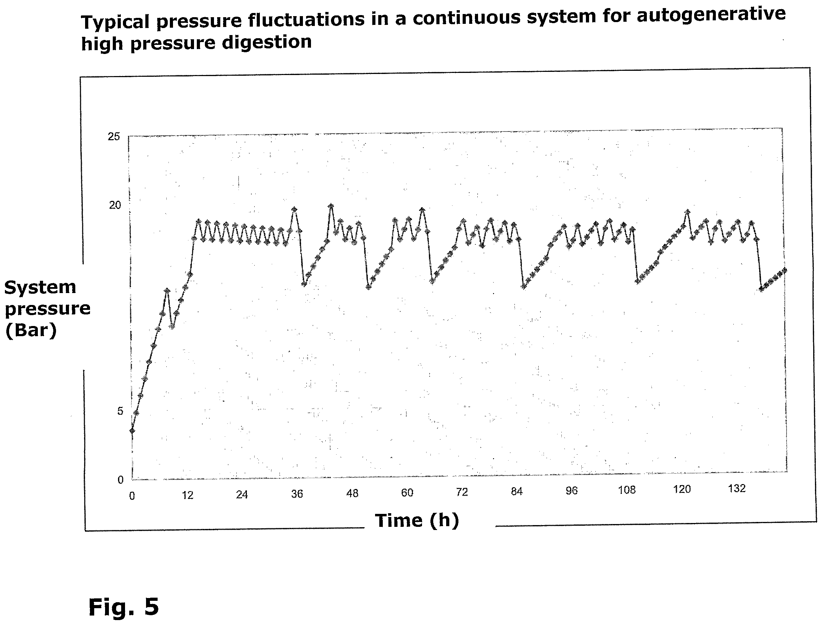

To system 1, an untreated flow 4 is supplied. The microorganisms convert this whereby biogas is formed. This purifies the supplied flow. The produced biogas can be put to use for other purposes such as driving a pneumatic motor/pump 22,24, the separation of methane and CO₂ according to Henry, drive the membrane separation process, drive a heat pump and dewatering of sludge.
Additionally and next to the caloric- or combustible energy of the produced biogas, it is possible to also use the elevated pressure of this biogas in converting into another form of usable energy, such as labour. Hereby system 1 is, in fact, used in more than one way as an energy generator. Preferably, both functions regarding energy generation based on the realized high pressure in the pressure vessel 8, are combined with the purifying and/or treatment of a supplied flow 4.
In addition to figure 1 (existing figure), the workings of the invention are described in figure 2. A perpetual cycle of 4 steps takes place, whereby in step 1, substratum is supplied through the gas pressure driven feed pump. In step 2 biogas is produced, in step 3 purified water (digestate) is removed and in step 4 other processes are driven (f.i. a heat pump or other users). Steps 1 till 4 can be repeated each on its own within a cycle and/or can be skipped and/or can be performed at random.
System 1 served as a basis for a number of experiments.
Some specifications of the system and materials/flows used are described, followed by some experimental results, as shown in figures 3-5.

The present invention is by no means restricted to the embodiment of preference, as described above. The requested rights are determined by the following claims within the scope of which many modifications are conceivable.

## Claims

1. System (1) for purifying/treatment of an organic material, waste and/or sewage under pressure, the system comprising:
- a sealable pressure vessel (8) provided with an inlet (25) to supply an influent (4) to be purified and/or treated whereby the pressure vessel is equipped to execute an anaerobe conversion of the influent in, amongst others, biogas;
- at least one exhaust (3,16) to drain off products from the pressure vessel;
- a control system (2) to control the purifying process;
- a pump (22) that is functionally connected to the inlet for supplying the influent to be purified and/or treated; and
- energy generating means to regulate and/or regenerate energy with, at least, a part of the produced biogas,
**characterized in that** the pump can be driven by the biogas from the pressure vessel, thereby using the biologically built up pressure and converting it into usable energy in the form of labor, and wherein the system is a closed energetic system, wherein the pump comprises a pneumatically driven pump, and wherein the control system is provided with regulating means to adjust the capacity of the pump to the pressure in the pressure vessel.

2. System according to claim 1, further comprising flow regulating means to control the flow through a sludge water membrane (12).

3. System according to claim 1 or 2, further comprising a membrane (6) provided in the pressure vessel for separation of at least a part of the produced biogas of the influent to be purified and/or treated.

4. System according to claim 3, wherein the membrane (6) is a selective gas membrane for removal of hydrogen-thiocarbonacid and/or carbon dioxide.

5. System according to one or more of the claims 1-4, wherein the inlet and at least one exhaust is provided with valves (18).

6. System according to one or more of the claims 1-5, further comprising a heat pump.

7. System according to one or more of the claims 1-6, further comprising a gas buffer to store the produced biogas.

8. Method for purification and/or treatment of organic waste and/or sewage containing the following steps:
- providing a system (1) according to one or more of the claims 1-7;
- supplying the system with an influent (4) to be purified and/or treated;
- separating and removing produced water from a residual flow;
- separating and removing dewatered sludge from a residual flow,
wherein the produced biogas is used to generate a closed energetic system.

9. Method for production of biogas, containing the following steps:
- providing a system (1) according to one or more of the claims 1-7;
- supplying the system with the influent (4) to be purified and/or treated;
- separating and removing the produced biogas of a residual flow.

10. Method according to claim 9, wherein the biogas comprises methane.

11. Method according to claim 8, 9 or 10, wherein carbon dioxide in the liquid phase is obtained by a pressure reduction and is used to counteract the pollution and/or fouling/and/or scaling of membranes.

## Patentansprüche

1. Anlage (1) zum Reinigen/Behandeln eines organischen Materials, Abfalls und/oder Abwassers unter Druck, umfassend:
- einen abdichtbaren Druckbehälter (8), der mit einem Einlass (25) zur Lieferung eines zu reinigenden und/oder zu behandelnden Zulaufs (4) vorgesehen ist, wobei der Druckbehälter ausgestattet ist, um eine anaerobe Umwandlung des Zulaufs unter anderem in Biogas durchzuführen,
- zumindest einen Auslass (3, 16), um Produkte aus dem Druckbehälter abzuleiten,
- ein Steuerungssystem (2) zum Steuern des Reinigungsprozesses,
- eine Pumpe (22) die funktionell mit dem Einlass zur Lieferung des zu reinigenden und/oder zu behandelnden Zulaufs verbunden ist,
- und eine Energieerzeugungseinrichtung zum Regulieren und/oder Regenerieren von Energie mit zumindest einem Teil des produzierten Biogases,
**dadurch gekennzeichnet, dass** die Pumpe durch das Biogas aus dem Druckbehälter betrieben werden kann, um dadurch den biologisch aufgebauten Druck zu nutzen und ihn in nutzbare Energie in Form von Arbeit umzusetzen,
und wobei die Anlage ein geschlossenes Energiesystem ist,
wobei die Pumpe eine pneumatisch betriebene Pumpe umfasst und wobei das Steuerungssystem mit Regulierungsmitteln versehen ist, um die Kapazität der Pumpe an den Druck in dem Druckbehälter anzupassen.

2. Anlage nach Anspruch 1, ferner umfassend eine Fließreguliereinrichtung zur Steuerung des Fließens durch eine Schlammwassermembran (12).

3. Anlage nach Anspruch 1 oder 2, ferner umfassend eine Membran (6), die in dem Druckbehälter zum Trennen von zumindest einem Teil des produzierten Biogases aus dem zu reinigenden und/oder zu behandelnden Zulauf vorgesehen ist.

4. Anlage nach Anspruch 3, wobei die Membran (6) eine selektive Gasmembran zum Entfernen von Wasserstoff-Thiocarbonazid und/oder Kohlendioxid ist.

5. Anlage nach einem oder mehreren der Ansprüche 1 bis 4, wobei der Einlass und zumindest ein Auslass mit Ventilen (18) versehen sind.

6. Anlage nach einem oder mehreren der Ansprüche 1 bis 5, ferner umfassend eine Wärmepumpe.

7. Anlage nach einem oder mehreren der Ansprüche 1 bis 6, ferner umfassend einen Gaspuffer zum Speichern des produzierten Biogases.

8. Verfahren zum Reinigen und/oder Behandeln von organischem Abfall und/oder Abwasser, enthaltend die folgenden Schritte:
- Bereitstellen einer Anlage (1) nach einem oder mehreren der Ansprüche 1 bis 7,
- Liefern eines zu reinigenden und/oder zu behandelnden Zulaufs (4) an die Anlage,
- Trennen und Entfernen von erzeugtem Wasser aus einem Restfluss,
- Trennen und Entfernen von entwässertem Schlamm aus einem Restfluss,
- wobei das produzierte Biogas zur Erzeugung eines geschlossenen Energiesystems genutzt wird.

9. Verfahren zum Herstellen von Biogas, enthaltend die folgenden Schritte:
- Bereitstellen einer Anlage (1) nach einem oder mehreren der Ansprüche 1 bis 7,
- Liefern des zu reinigenden und/oder zu behandelnden Zulaufs (4) an die Anlage,
- Trennen und Entfernen des produzierten Biogases aus einem Restfluss.

10. Verfahren nach Anspruch 9, wobei das Biogas Methan umfasst.

11. Verfahren nach Anspruch 8, 9 oder 10, wobei Kohlendioxid in der flüssigen Phase durch eine Druckreduzierung erhalten und dazu genutzt wird, dem Verschmutzen und/oder Verunreinigen und/oder Verkrusten von Membranen entgegenzuwirken.

## Revendications

1. Système (1) pour purifier et traiter un matériau organique, des déchets et/ou des eaux usées sous pression, le système comprenant :
- un récipient sous pression scellable (8) doté d'une entrée (25) pour fournir un effluent (4) à purifier et/ou traiter, selon lequel le récipient sous pression est équipé pour exécuter une conversion anaérobie de l'effluent en, entre autres, un biogaz ;
- au moins un échappement (3 16) pour évacuer des produits du récipient sous pression ;
- un système de commande (2) pour commander le processus de purification ;
- une pompe (22) qui est reliée fonctionnellement à l'entrée pour fournir l'effluent à purifier et/ou traiter ; et
- des moyens de génération d'énergie pour réguler et/ou régénérer l'énergie avec au moins une partie du biogaz produit,
**caractérisé en ce que** la pompe peut être entraînée par le biogaz venant du récipient sous pression, en utilisant ainsi la pression produite biologiquement et en la convertissant en énergie utilisable sous forme de travail, et dans lequel le système est un système énergétique fermé, dans lequel la pompe comprend une pompe entraînée par voie pneumatique et dans lequel le système de commande est doté de moyens de régulation pour ajuster la capacité de la pompe à la pression dans le récipient sous pression.

2. Système selon la revendication 1, comprenant en outre des moyens de régulation de flux pour contrôler le flux via une membrane d'eau de boues (12).

3. Système selon la revendication 1 ou 2, comprenant en outre une membrane (6) prévue dans le récipient sous pression pour la séparation d'au moins une partie du biogaz produit de l'effluent à purifier et/ou traiter.

4. Système selon la revendication 3, dans lequel la membrane (6) est une membrane de gaz sélective pour le retrait d'hydrogène-thio-acide de carbone et/ou de dioxyde de carbone.

5. Système selon l'une ou plusieurs des revendications 1 à 4, dans lequel l'entrée et au moins un échappement sont dotés de valves (18).

6. Système selon l'une ou plusieurs des revendications 1 à 5, comprenant en outre une pompe à chaleur.

7. Système selon l'une ou plusieurs des revendications 1 à 6, comprenant en outre un réservoir tampon de gaz pour stocker le biogaz produit.

8. Procédé pour purifier et/ou traiter des déchets organiques et/ou eaux usées contenant les étapes suivantes :
- fournir un système (1) selon l'une ou plusieurs des revendications 1 à 7 ;
- fournir au système un effluent (4) à purifier et/ou à traiter ;
- séparer et retirer l'eau produite d'un flux résiduaire ;
- séparer et retirer la boue déshydratée d'un flux résiduaire,
dans lequel le biogaz produit est utilisé pour générer un système énergétique fermé.

9. Procédé de production de biogaz contenant les étapes suivantes :
- fournir un système (1) selon une ou plusieurs des revendications 1 à 7 ;
- fournir au système l'effluent (4) à purifier et/ou à traiter ;
- séparer et retirer le biogaz produit d'un flux résiduaire.

10. Procédé selon la revendication 9, dans lequel le biogaz comprend du méthane.

11. Procédé selon la revendication 8, 9 ou 10, dans lequel du dioxyde de carbone en phase liquide est obtenu par réduction de pression et est utilisé pour combattre la pollution et/ou l'encrassement et/ou l'entartrage de membranes.
